# NEW EUROPEAN PATENT SPECIFICATION

(11) **EP 2 666 517 B2**
(45) Date of publication and mention of the opposition decision: **01.01.2020**
(45) Mention of the grant of the patent: 13.07.2016
(21) Application number: 13168658.6
(22) Date of filing: 22.05.2013
(51) Int. Cl.: A61Q 11/00, A61K 8/73, A61K 8/34, A61K 8/44, A61K 8/81, A61K 8/19

(54) **Anticaries formulation**
Antikariesformulierung
Formulation anti-carie

(30) Priority: 22.05.2012 IT MI20120883
(43) Date of publication of application: 27.11.2013
(73) Proprietor: Quattroti Dentech S.R.L., 21040 Cislago (VA) (IT)
(72) Inventor: Turchetti, Mauro, I-21047 Saronno (VA) (IT)
(74) Representative: Allaix, Roberto

(56) References cited:
- EP-A1- 0 444 492
- WO-A1-2006/013081
- US-A1- 2010 022 471
- RODRIGUES SV ET AL: "Hyaluronan-containing mouthwash as an adjunctive plaque-control agent", ORAL HEALTH & PREVENTIVE DENTISTRY, QUINTESSENCE PUBLISHING CO. LTD, UK, vol. 8, no. 4, 1 January 2010 (2010-01-01) , pages 389-394, XP009167760, ISSN: 1602-1622
- SHIBASAKI K-I ET AL: "EFFECTS OF LOW MOLECULAR CHITOSAN ON PH CHANGES IN HUMAN DENTAL PLAQUE", TOKYO DENTAL COLLEGE BULLETIN, CHIBA, JP, vol. 35, 1 February 1994 (1994-02-01), pages 33-39, XP009053879,

## Description

The present invention refers to a new oral formulation for preventing the dental caries.

The main causes of the caries formation are the bacteria present in the dental plaque. The presence of food residues in the oral cavity promotes, in fact, the growth in the plaque of Gram positive "acidic" bacteria, which reduce the pH, and cause the caries formation.

Specifically, the dental caries formation caused by such bacteria is a two-stage process. In the first stage, the bacteria present in the plaque metabolize, by fermentation, the carbohydrates present in it, causing the formation of acids. In the second stage, the formed acids demineralize the enamel, dentin and/or the cement of the teeth, and form a lesion or a caries cavity in the crown or root of the tooth (Miller W.D. (1890) "Micro-organisms of the human mouth," Reprinted 1973, Karger, Basel).

The diet is the primary source of fermentable carbohydrates, mainly glucose, metabolized by the bacteria in the first stage of the above-mentioned process.

Therefore, after the intake of food, if the buffering action of the saliva is not sufficient to compensate for the production of acids, the pH of the plaque lowers from the physiological values of 6-6.5 to values of 4-5. For pH values less than 5.5, the enamel starts dissolving and the caries forms.

A gradual return to physiological values of the pH occurs thanks to buffer systems, particularly the phosphate and bicarbonate systems, physiologically present in the plaque and saliva (Jenkins, GN The physiology of the mouth, Blackwell, 1970). Among these systems, the most important is the bicarbonate system. The bicarbonate concentration in saliva is tied to the formation rate of the same. As the saliva formation rate increases, also the production of the bicarbonate formed by the metabolism of the saliva glands increases. Therefore, in a physiological state, when food is intaken, the consequent increase in the saliva flow leads to a production of bicarbonate buffering the acid formation. However, sometimes such production is not capable to adequately compensate the formation of acids since the above described events occur inside the dental plaque, and consequently in order to neutralize the cariogenic effect, the bicarbonate must diffuse from saliva to the plaque and neutralize *in situ* the acids formed by the fermentated food.

One of the main bacteria causing the caries formation is *Streptococcus mutans* (Daculsi G, Le Geros RZ, Jean, A & Kerebel, B Possible physico-chemical processes in human dentine caries, J Dent Res. 1987, v. 66, 1356-1359; Edwardsson, S., Bacteriology of dentin caries, Dentine and dentine reactions in the oral cavity, Ed. Thylstrup A, Leach SA & Qvist V. IRL Press, 1987).

This bacterium is capable of producing, from fermentable sugars, and particularly saccharose, glucans and fructans which promote the adhesion of bacteria to the mouth surface. A plaque containing high quantities of *S. mutans* is highly cariogenic because it rapidly metabolizes sugars, bringing the pH to values inferior to the critical one of 5.5.

In the recent years, xylitol has aroused the interest due to its anticariogenic action, which is capable of interfering with the *S. mutans* metabolism, thus reducing the concentration of this bacterium in the dental plaque.

Several products preventing caries formation, generally sold as mouthwash are commercially available. However, despite this, the dental caries incidence is still very high, mostly in individuals having salivation problems.

Further, the majority of the sold products contains preservatives such as parabens, organic acids, chlorexidine or imidazolidinyl urea, whose presence is undesired. For example, chlorexidine, used as preservative in many commercially available mouthwashes, has side effects such as, for example, an altered sense of taste, a dark shaded teeth and tongue, and a delayed wound healing process.

Therefore, there is the need of make available new formulations capable of effectively preventing caries formation. Moreover, there is the need of developing liquid anticaries formulations which do not contain preservatives.

### SUMMARY OF THE INVENTION

The Applicant has found that an aqueous solution having a pH comprised between 7 and 10 and comprising sodium bicarbonate, xylitol, and a mucoadhesive polymer, presents a surprisingly high anticaries efficacy.

Further, the Application has surprisingly found that when to the above components, are also added a chelating agent, a detergent surfactant and at least a monomeric polyol, the resulting formulation does not require the traditional preservatives.

Therefore, object of the present invention is an aqueous liquid oral formulation in the form of a mouthwash or in a nebulizable form having a pH between 7 and 10 and comprising sodium bicarbonate, xylitol, a mucoadhesive polymer and, optionally, a chelating agent, a detergent surfactant and at least a monomeric polyol. Moreover, a further object of the present invention is the use of the same in the oral hygiene for delaying or preventing caries formation.

One or more of the objectives that will be better explained during the following description are substantially met by a new formulation for preventing the dental caries according to one or more of the attached claims.

Aspects of the invention will be described in the following.

In a first aspect, it is provided an aqueous liquid oral formulation in the form of a mouthwash or in a nebulizable form having a pH between 7 and 10, and comprising xylitol, a bicarbonate salt and a mucoadhesive polymer.

In a second aspect according to the preceding aspect, the polymer is selected from a group constituted by polyacrylic acid, hydroxypropyl cellulose, hydroxypropyl methyl cellulose, hydroxyethyl cellulose, polyvinylpyrrolidone, polymethacrylate derivatives, hyaluronic acid and derivatives thereof, chitosan and mixtures thereof.

In a third aspect according to anyone of the preceding aspects, the polymer is sodium hyaluronate.

In a fourth aspect according to anyone of the preceding aspects, the polymer is present in the formulation of the invention in an amount comprised between 0.02% and 0.1% (w/w).

In a fifth aspect according to anyone of the preceding aspects, xylitol is contained in the formulation in an amount comprised between 8% and 20% (w/w).

In a sixth aspect according to anyone of the preceding aspects, the bicarbonate salt is contained in the formulation in an amount comprised between 1% and 2.5% (w/w).

In a seventh aspect according to anyone of the preceding aspects, the formulation further comprises at least a chelating agent.

In an eighth aspect according to anyone of the preceding aspects, the formulation further comprises at least one surfactant agent.

In a ninth aspect according to anyone of the preceding aspects, the formulation further comprises at least a monomeric polyol.

In a tenth aspect according to anyone of the seventh-ninth aspects, the chelating agent is tetra sodium EDTA.

In an eleventh aspect according to anyone of the seventh-tenth aspects, the chelating agent is present in an amount comprised between 0.02% and 1% (w/w).

In a twelfth aspect according to anyone of the eight-eleventh aspects, the surfactant agent is selected from sodium lauroyl sarcosinate, sodium lauryl sulphate, poloxamer 407 and polysorbate 20.

In a thirteenth aspect according to anyone of the ninth-twelfth aspects, the monomeric polyol is selected from glycerine and glycol.

In a fourteenth aspect according to anyone of the ninth-thirteenth aspects, the monomeric polyol is present in the formulation in an amount comprised between 10 and 35% (w/w).

In a fifteenth aspect according to anyone of the preceding aspects, the formulation comprises xylitol, in an amount comprised between 10 and 15% (w/w), sodium bicarbonate, in an amount comprised between 1 and 2% (w/w), sodium hyaluronate HMW, in an amount comprised between 0.02 and 0.08% (w/w), glycerine, in an amount comprised between 15 and 25% (w/w), butylene glycol, in an amount comprised between 8 and 15% (w/w), sodium lauroyl sarcosinate, in an amount comprised between 0.2 and 0.8% (w/w), tetrasodium EDTA, in an amount comprised between 0.02 and 0.08% (w/w), sodium hydroxide (30% water solution) in an amount sufficient to bring the pH to between 7 and 10, purified water up to the desired volume.

In a sixteenth aspect according to the preceding aspect, the formulation comprises 12% (w/w) xylitol, 1.5% (w/w) sodium bicarbonate, 0.05% (w/w) sodium hyaluronate HMW, 20% (w/w) glycerine, 10% (w/w) butylene glycol, 0.5% (w/w) sodium lauroyl sarcosinate, 0.05% tetrasodium EDTA (w/w); sodium hydroxide (30% water solution) in sufficient amount to bring the pH to between 7 and 10, purified water to the desired volume.

In a seventeenth aspect according to anyone of the first-fourteenth aspects, the formulation comprises xylitol, in an amount comprised between 12 and 18% (w/w), sodium bicarbonate, in an amount comprised between 1.5 and 2.5 (w/w), sodium hyaluronate HMW, in an amount comprised between 0.02 and 0.08% (w/w), glycerine, in an amount comprised between 10 and 20% (w/w), sodium lauroyl sarcosinate in an amount comprised between 0.5% and 1.5% (w/w), Tetrasodium EDTA, in an amount comprised between 0.02 and 0.08% (w/w), trimethylglycine, in an amount comprised between 8 and 12% (w/w), sodium hydroxide (30% water solution) in an amount sufficient to bring the pH to between 7 and 10, purified water up to the desired volume.

In an eighteenth aspect according to the preceding aspect, the formulation comprises 15% (w/w) xylitol, 2% (w/w) sodium bicarbonate, 0.05% (w/w) sodium hyaluronate HMW, 15% (w/w) glycerine, 1% (w/w) sodium lauroyl sarcosinate, 0.05% (w/w) tetrasodium EDTA, 10% (w/w) trimethylglycine, sodium hydroxide (30% water solution) in an amount sufficient to bring the pH to between 7 and 10, purified water up to the desired volume.

In a nineteenth aspect according to anyone of the preceding aspects, the formulation is for use in the oral hygiene.

In a twentieth aspect according to the preceding aspect, the formulation is for an use in the reduction or prevention of the formation of dental caries.

### DETAILED DESCRIPTION OF THE DRAWINGS

Figure 1 illustrates the variation of the average values of the pH of artificial saliva, obtained by adding increasing quantities of the Formulation 1 of example 1.
Figure 2 shows the trend of the average salivary pH of all the analyzed subjects following an administration of 10 ml of a 10% glucosated solution without (lower line, grey) or with (upper line, black) the tested product, the Formulation 1 described in Example 1.
Figure 3 shows the distribution of the averages of the permanence times at pH<6 during a pH monitoring time of 40 minutes following an administration of 10 ml of a 10% glucosated solution without (min) or with (min_c) the Formulation 1 described in Example 1.

### DETAILED DESCRIPTION OF THE INVENTION

A first object of the present invention is an aqueous liquid formulation having a pH between 7 and 10 and comprising xylitol, a bicarbonate salt, preferably selected from lithium bicarbonate, potassium bicarbonate, arginina bicarbonate and sodium bicarbonate, among which the most preferred is the sodium bicarbonate, and a mucoadhesive polymer.

According to the present invention, the term "mucoadhesive polymer" means a polymer capable of adhering, through the formation of chemical and/or physical bonds, to the buccal mucosa. Preferably, said polymer is selected from a group constituted by polyacrylic acid, hydroxypropyl cellulose, hydroxypropyl methyl cellulose, hydroxyethyl cellulose, polyvinylpyrrolidone, hyaluronic acid and derivatives thereof, chitosan and mixtures thereof. A particularly preferred mucoadhesive polymer is sodium hyaluronate. Preferably, the mucoadhesive polymer is present in the formulation of the invention in an amount between 0.02% and 0.1% (w/w), more preferably between 0.04% and 0.07% (w/w) and still more preferably of 0.05% (w/w).

Preferably said xylitol is contained in the formulation of the invention in an amount between 8% and 20% (w/w), more preferably between 10% and 17% (w/w).

Preferably, said bicarbonate salt is contained in the formulation in an amount between 1% and 2.5% (w/w), more preferably between 1.3% and 2.2% (w/w).

According to a particularly preferred embodiment of said invention, said formulation further comprises at least a chelating agent, preferably selected from EDTA and its salts and gluconic acid and its salts, and a surfactant agent. The Applicant has discovered that the addition of these further components to the formulation enables to obtain a high microbiologic quality without the need to add standard preservatives.

Preferably, the chelating agent is a salt of EDTA, selected from di-, tri- or tetrasodium EDTA, among which tetrasodium EDTA is particularly preferred. Preferably, the chelating agent is present in the inventive formulation in an amount between 0.02% and 1% (w/w), more preferably between 0.04% and 0.07% (w/w), and still more preferably of 0.05% (w/w).

Preferably, said surfactant agent is selected from sodium lauroyl sarcosinate, sodium lauryl sulphate, sodium lauryl ether sulphate, poloxamer 407 and polysorbate 20, among which sodium lauryl sarcosinate is particularly preferred. Preferably, the inventive formulation contains the surfactant agent in an amount between 0.1% and 1.5% (w/w), more preferably between 0.3% (w/w) and 1.2% (w/w), still more preferably 0.5% or 1% (w/w).

According to a further preferred embodiment of the invention, said formulation further contains at least a further monomeric polyol besides xylitol, selected from glycerine and/or glycols. The preferred glycols are propylene glycol, butylene glycol, pentylene glycol, hexylene glycol, undecylenic glycol, decylenic glycol, among which propylene glycol is particularly preferred.

Preferably, said polyols are present in the inventive formulation in an amount comprised between 10 and 35% (w/w). Preferably, said polyols comprise glycerine in an amount comprised between 10 and 25% (w/w) and at least a glycol in an amount comprised between 0 and 15% (w/w).

Particularly preferred formulations according to the invention comprise the following components, in weight/weight:
xylitol, in an amount between 10 and 15%, preferably 12%; sodium bicarbonate, in an amount between 1 and 2%, preferably of 1.5%; sodium hyaluronate HMW, in an amount between 0.02 and 0.08%, preferably of 0.05%;
glycerine, in an amount between 15 and 25%, preferably 20%; butylene glycol, in an amount between 8 and 15%, preferably of 10%; sodium lauryl sarcosinate, in an amount between 0.2 and 0.8%, preferably of 0.5%; tetrasodium EDTA, in an amount between 0.02 and 0.08%, preferably of 0.05%; sodium hydroxide (30% aqueous solution) in an amount sufficient to bring the pH to between 7 and 10; purified water up to the desired volume, or
xylitol, in an amount between 12 and 18%, preferably 15%; sodium bicarbonate, in an amount comprised between 1.5 and 2.5, preferably 2%; sodium hyaluronate HMW in an amount between 0.02 and 0.08%, preferably of 0.05%;
glycerine, in an amount comprised between 10 and 20%, preferably 15%; sodium lauryl sarcosinate in an amount comprised between 0.5% and 1.5%, preferably of 1%; tetrasodium EDTA, in an amount between 0.02 and 0.08&, preferably of 0.05%; trimethyl glycine, in an amount between 8 and 12%, preferably of 10%; sodium hydrosside (30% aqueous solution) in an amount sufficient to bring the pH to between 7 and 10; purified water up to the desired volume.

To said formulations, further excipients can be added, suitable for formulations for oral use in order to improve their organoleptic and/or formulative characteristics. Said excipients are well known from the prior art.

As it will be clearly explained in the following experimental section, the inventive formulation has surprising properties in the caries prevention. Particularly, the present formulation shows an 85% reduction of the Streptococcus Mutans, which is considered the main etiologic agent of the dental caries. Further, the inventive formulation effectively and continuously prevents a pH reduction occurring due to the food intake, counteracting in such a way the enamel dissolution which promotes the caries formation.

Therefore, the present invention has, as a further object, the use of said formulation for the oral hygiene in order to delay and/or prevent the dental caries. The use of the present invention formulation for this object is performed by a topic administration at the oral cavity.

A particularly preferred formulation of the invention has the form of a mouthwash. Preferably, said mouthwash is in a form suitable to be nebulised in the oral cavity. To this end, the formulation is introduced in an suitable container, provided with a pump for nebulising it.

The present invention will be described in more details by the following non limiting examples.

### Example 1:

Formulations having the following qualitative-quantitative composition are prepared by using the below procedures:

**Formulation 1**

| | % |
|---|---|
| Xylitol | 12.000 |
| Sodium bicarbonate | 1.500 |
| Sodium hyaluronate HMW | 0.050 |
| Glycerine | 20.000 |
| Butylene glycol | 10.000 |
| Sodium lauryl sarcosinate | 0.500 |
| Tetrasodium EDTA | 0.050 |
| Aroma | 0.800 |
| Sodium hydroxide (30% aqueous q.b. for a pH solution) | between 7 and 10 |
| Purified water | Up to 100 |

**Formulation 2**

| | % |
|---|---|
| Xylitol | 15.000 |
| Sodium bicarbonate | 2.000 |
| Sodium hyaluronate HMW | 0.050 |
| Glycerine | 15.000 |
| Trimethylglycine | 10.000 |
| Sodium lauryl sarcosinate | 1.000 |
| Tetrasodium EDTA | 0.050 |
| Aroma | 0.500 |
| Sodium hydroxide (30% aqueous q.b. for a pH solution) | between 7 and 10 |
| Purified water | Up to 100 |

Hyaluronic acid is dissolved in water, to the solution are then sodium bicarbonate, glycerine, xylitol, butilene glycol or trimethylglycine, tetrasodium EDTA, an aroma premixed with sodium lauryl sarcosinate are added. If necessary, sodium hydroxide is added so the pH is brought to the desired value.

### Example 2

### Organoleptic and chemical-physical characteristics

The organolpetic and chemical-physical characteristics of the two formulations of example 1 have been analyzed with the results, for both formulations, listed in the following table:

| | |
|---|---|
| Appearance (at 25°C) | Clear solution |
| Colour (at 25°C) | Colourless |
| Odour (at 25°C) | Typical |
| pH (at 25°C) Ph Eur 2.2.3 current edition | 7.00 - 10.00 |
| Specific Gravity (as it @ 25°C) Ph Eur 2.2.3 current edition | 0.900 - 1.200 g/ml |
| TAMC Ph Eur 2.6.12 Ed 6.5 page 4769 | Max 10² micro-organisms for gr or ml |
| TYMC Ph Eur 2.6.12 Ed 6.5 page 4769 | Max 10¹ micro-organisms for gr o ml |
| Pseudomonas Aeruginosa Ph Eur 2.6.13 Ed 6.5 page 4773 | Absent in 1 g or ml |
| Staphylococcus Aureus Ph Eur 2.6.13 Ed 6.5 page 4773 | Absent in 1 g or ml |
| Candida Albicans Ph Eur 2.6.13 Ed 6.5 page 4773 | Absent in 1 g or ml |
| Escherichia Coli Ph Eur 2.6.13 Ed 6.5 page 4773 | Absent in 1 g or ml |

### Example 3

### Studies about the efficacy

The efficacy of the formulation 1 of Example 1 has been evaluated by two different *in vitro* studies intended to analyze the buffering and antibacterial properties of the formulations:

### a. Analysis of the buffering capacity

30 ml of artificial saliva prepared according to UNI EN 12868:2002 are transferred into a 50 ml becker, wherein 2.1 ml (corresponding to 70 µl of the formulation to be tested for ml of artificial saliva) of the formulation to be tested are added for 5 consecutive times until a total quantity of the formulation to be tested equivalent to 350 µl for 1 ml of artificial saliva are reached,. After each one of the 5 additions, the pH value is measured.

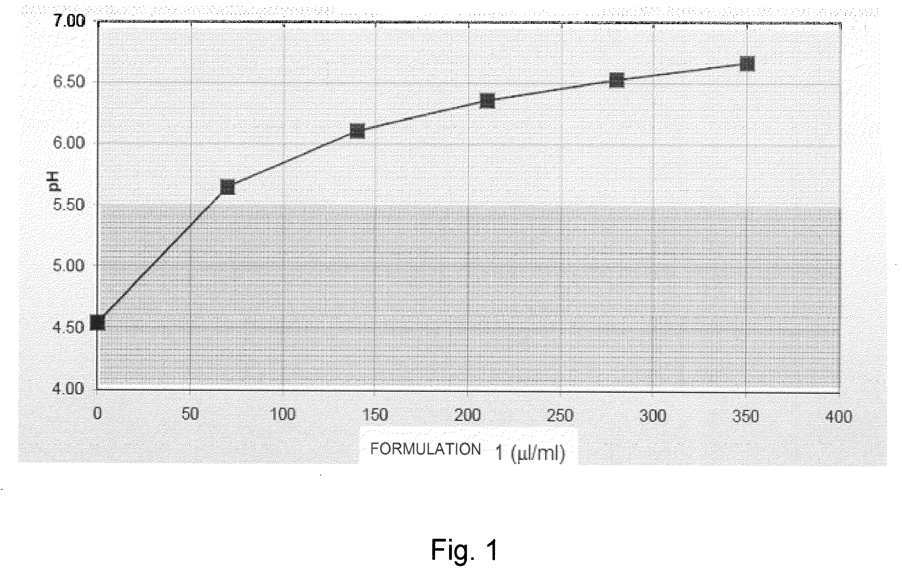

The experiment has been performed in triplicate and the average value of pH obtained at each addition in the three tests is calculated. Figure 1 shows the obtained data, that demonstrate that the addition of 140 µl of the product increase the saliva pH to a value above 5.5, a critical value under which the enamel starts dissolving and the caries forms.

### a. Analysis of the antibacterial capacity

9.5 ml of formulation 1 are added to 0.5 ml of a suspension of *Streptococcus mutans* containing ≥ 10⁸ CFU/ml for different time intervals. After an exposure, part of the suspension is transferred to a neutralizer and is tested for verifying the number of survived microorganisms.

The obtained results are reported in the following table:

| **Test Substance: DENTAL SPRAY DM019 pr 22 Batch: LCOXVIII/45** | | | | | | |
|---|---|---|---|---|---|---|
| **Test Organism** | **Exposure Time** | **CFU/mL in Test Population Control* (Log₁₀)** | **CFU/mL of Survivors*** | **Log₁₀ Survivors** | **Percent Reduction** | **Log₁₀ Reduction** |
| *Streptococcus mutans* (ATCC 25175) | 5 minutes | 5.5 x 10⁵ (5.74) | 7.8 x 10⁴ | 4.89 | 85.8% | 0.85 |
| | 15 minutes | | 2.74 x 10⁴ | 4.44 | 95.0% | 1.30 |
| | 25 minutes | | 1.28 x 10⁴ | 4.11 | 97.7% | 1.63 |
| | 40 minutes | | 7.7 x 10³ | 3.89 | 98.6% | 1.85 |

The obtained data show that in the conditions provided by the protocol ASTM E2315-03, the product is capable of causing an 85% reduction of Streptococcus Mutans, which is considered the main etiologic agent of the dental caries, already after a contact time of 5 minutes at a temperature of 37°C, in the presence of 5% of FBS (Fetal Bovine Serum).

### Example 4

A sample of 30 adult subjects was selected among the patients of the Odontoiatric clinic of the Insubria Study University. The sample of the selected population showed good health oral conditions (absence of caries, parodontid, lesions of oral mucosas) and a negative anamnesis for any type of systemic pathology.

The salivary pH analysis was performed using a 2-channel portable pH meter (pH-day 2; Menfis bioMedica S.r.l.) provided with a single-use antimony probe, already used in other studies on salivary pH. The apparatus records in continuous the pH value in a sampling period from 1 to 6 seconds, with a resolution of 0.1 pH and a range from 0.1 to 14 pH. The probe used was calibrated before each exams with buffer solutions at pH 1 and pH 7 according to instructions provided by the manufacturer.

All the measurements were performed during the morning hours: the subjects arrived without having eaten and without having done any oral hygiene for at least two hours. The pH measurement was been performed in the lower furnice in the first molars area.

Day 1, the subjects washed their mouth with 10 ml of 10% glucosated solution and the pH was monitored for 40 minutes.

Day 2, the same experimental procedure was repeated, with the addition of three 3 doses of the mucoadhesive spray of formulation 1, described in Example 1, at the buccal mucosa.

Subsequently, the values of the averages and medians of the obtained pH values, with the corresponding standard deviations, in the subjects during the two tests time were processed.

Moreover, it was calculated for both groups the pH permanence time at values inferior to 6 and the time percentage wherein the pH was below such value during the total times of 40 minutes in which the pH was monitored. The results were compared with the Wilcoxon-Mann-Whitney test for evaluating the significance of the found differences.

### Results:

A certain individual variability in the recorded pH values was determined, the initial values were in fact comprised in a range between 5.9 and 7.5 and the downward or upward pH variations showed in each patient different amplitudes. This result can be understood by considering that the basal pH values are different from a subject to another and by considering the different individual answer to the glucogenic challenge in relation to the acidophilic bacterial load present and to the salivary buffering capacity of the subject.

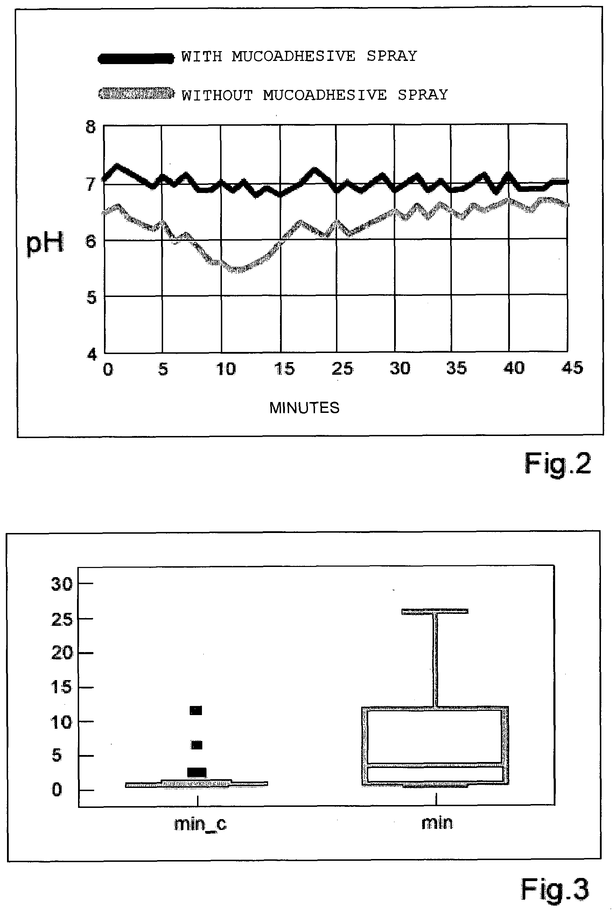

By using the results calculated as an average, minute by minute during the 40 minutes monitored, the graphic of figure 2 was obtained, showing the average salivary pH trend in all the subjects analyzed with (upper line, black) and without (lower line, grey) the tested product.

The distribution of the averages of the permanence times at pH<6 during the 40 minutes monitoring the pH are instead shown in figure 3.

The obtained results show that, applying 3 doses of the tested mucoadhesive spray on the buccal mucosa, it was obtained a salivary pH values increase, observable in the average values during the 40 minutes (p=0.0016)(min_c).

Such pH variations are clinically relevant, because the enamel demineralization phenomena have been observed near a pH value of 5.7.

Moreover, in the presence of the product to be tested, it is also observed an increase of the permanence time of the pH at values less than 6 (p=0.0001).

Therefore, the obtained results show that the mucoadhesive effect enables a progressive release of the bicarbonate and a prolonged increase of the buffering capacity of the saliva.

## Claims

1. An aqueous liquid oral formulation having a pH comprised between pH 7 and 10 and comprising xylitol, a bicarbonate salt and a mucoadhesive polymer, in particular selected from a group constituted by polyacrylic acid, hydroxypropyl cellulose, hydroxypropyl methyl cellulose, hydroxyethyl cellulose, polyvinylpyrrolidone, polymethacrylate derivatives, hyaluronic acid and derivatives thereof, chitosan and mixtures thereof, wherein said formulation is in a the form of a mouthwash or in a nebulizable form.

2. The formulation of claim 1, wherein the polymer is sodium hyaluronate.

3. The formulation of claim 1 or 2, wherein the polymer is present in the formulation of the invention in an amount comprised between 0.02% and 0.1% (w/w).

4. The formulation of claims 1 to 3, wherein the xylitol is contained in the formulation in an amount comprised between 8% and 20% (w/w).

5. The formulation of claims 1 to 4, wherein the bicarbonate salt is contained in the formulation in an amount comprised between 1% and 2.5% (w/w)

6. The formulation of claims 1 to 5, further comprising at least a chelating agent, a surfactant agent, in particular selected from sodium lauroyl sarcosinate, sodium lauryl sulphate, sodium lauryl ether sulphate, poloxamer 407 and polysorbate 20 and at least a monomeric polyol, in particular selected from glycerine and a glycol.

7. The formulation of claim 6, wherein the chelating agent is tetrasodium EDTA.

8. The formulation of claim 6 or 7, wherein said chelating agent is present in an amount comprised between 0.02% and 1% (w/w).

9. The formulation of claims from 6 to 8, wherein said monomeric polyol is present in the formulation in an amount comprised between 10 and 35% (w/w).

10. The formulation of claims 1 to 9, comprising xylitol, in an amount comprised between 10 and 15% (w/w), sodium bicarbonate, in an amount comprised between 1 and 2% (w/w), sodium hyaluronate HMW, in an amount comprised between 0.02 and 0.08% (w/w), glycerine, in an amount comprised between 15 and 25% (w/w), butylene glycol, in an amount comprised between 8 and 15% (w/w), sodium lauroyl sarcosinate, in an amount comprised between 0.2 and 0.8% (w/w), tetrasodium EDTA, in an amount comprised between 0.02 and 0.08% (w/w), sodium hydroxide in an amount sufficient to bring the pH to between 7 and 10, purified water up to the desired volume.

11. The formulation of claim 10, comprising 12% (w/w) xylitol, 1.5% (w/w) sodium bicarbonate, 0.05% (w/w) sodium hyaluronate HMW, 20% (w/w), glycerine, 10% (w/w) butylene glycol, 0.5% (w/w) sodium lauroyl sarcosinate, 0.05% tetrasodium EDTA (w/w); sodium hydroxide in sufficient amount to bring the pH to between 7 and 10, purified water to the desired volume.

12. The formulation of claims 1 to 9, comprising xylitol, in an amount comprised between 12 and 18% (w/w), sodium bicarbonate, in an amount comprised between 1.5 and 2.5 (w/w), sodium hyaluronate HMW, in an amount comprised between 0.02 and 0.08% (w/w), glycerine, in an amount comprised between 10 and 20% (w/w), sodium lauroyl sarcosinate in an amount comprised between 0.5% and 1.5% (w/w), Tetrasodium EDTA, in an amount comprised between 0.02 and 0.08% (w/w), trimethylglycine, in an amount comprised between 8 and 12% (w/w), sodium hydroxide in an amount sufficient to bring the pH to between 7 and 10, purified water up to the desired volume.

13. The formulation of claim 12, comprising 15% (w/w) xylitol, 2% (w/w) sodium bicarbonate, 0.05% (w/w) sodium hyaluronate HMW, 15% (w/w) glycerine, 1% (w/w) sodium lauroyl sarcosinate, 0.05% (w/w) tetrasodium EDTA, 10% (w/w) trimethylglycine, sodium hydroxide in an amount sufficient to bring the pH to between 7 and 10, purified water up to the desired volume.

14. The formulation of claims 1 to 13, for oral hygiene use, in particular for the reduction or prevention of the formation of dental caries.

## Patentansprüche

1. Wässrige, flüssige, orale Formulierung mit einem pH-Wert zwischen 7 und 10, umfassend Xylitol, ein Bicarbonatsalz und ein mucoadhäsives Polymer, insbesondere ausgewählt aus einer Gruppe bestehend aus Polyacrylsäure, Hydroxypropylcellulose, Hydroxypropylmethylcellulose, Hydroxyethylcellulose, Polyvinylpyrrolidon, Polymethacrylatderivate, Hyaluronsäure und deren Derivate, Chitosan und Mischungen davon, wobei die Formulierung in Form einer Mundspülung oder einer sprühenden Form vorliegt.

2. Formulierung nach Anspruch 1, wobei das Polymer Natriumhyaluronat ist.

3. Formulierung nach Anspruch 1 oder 2, wobei das Polymer in der erfindungsgemäßen Formulierung in einer Menge zwischen 0,02 % und 0,1 % (p/p) vorliegt.

4. Formulierung nach den Ansprüchen 1 bis 3, wobei das Xylitol in der Formulierung in einer Menge zwischen 8 % und 20% (p/p) enthalten ist.

5. Formulierung nach den Ansprüchen 1 bis 4, wobei das Bicarbonatsalz in der Formulierung in einer Menge zwischen 1 % und 2,5 % (p/p) enthalten ist.

6. Formulierung nach den Ansprüchen 1 bis 5, ferner umfassend mindestens einen Chelatbildner, ein Tensid, insbesondere ausgewählt aus Natriumlauroylsarcosinat, Natiumlaurylsulfat, Natriumlaurylethersulfat, Poloxamer 407 und Polysorbat 20 und mindestens einen monomeren Polyol, insbesondere ausgewählt aus Glycerin und einem Glykol.

7. Formulierung nach Anspruch 6, wobei der Chelatbildner Tetranatrium EDTA ist.

8. Formulierung nach Anspruch 7 oder 8, wobei der Chelatbildner in einer Menge zwischen 0,02 % und 1 % (p/p) vorliegt.

9. Formulierung nach den Ansprüchen 6 bis 8, wobei das monomere Polyol in der Formulierung in einer Menge zwischen 10 und 35 % (p/p) vorliegt.

10. Formulierung nach den Ansprüchen 1 bis 9, umfassend Xylitol in einer Menge zwischen 10 und 15 % (p/p), Natriumbicarbonat in einer Menge zwischen 1 und 2 % (p/p), Natriumhyaluronat HMW in einer Menge zwischen 0,02 und 0,08 % (p/p), Glycerin in einer Menge zwischen 15 und 25 % (p/p), Butylenglykol in einer Menge zwischen 8 und 15 % (p/p), Natriumlauroylsarcosinat in einer Menge zwischen 0,2 und 0,8 % (p/p), Tetranatrium EDTA in einer Menge zwischen 0,02 und 0,08 % (p/p), Natriumhydroxid in einer Menge, die ausreicht, um den pH-Wert zwischen 7 und 10 einzustellen, gereinigtes Wasser bis zum gewünschten Volumen.

11. Formulierung nach Anspruch 10, umfassend 12 % (p/p) Xylitol, 1,5 % (p/p) Natriumbicarbonat, 0,05 % p/p) Natriumhyaluronat HMW, 20 % (p/p) Glycerin, 10 % (p/p) Butylenglykol, 0,5 % (p/p) Natriumlauroylsarcosinat, 0,05 % (p/p) Tetranatrium EDTA; Natriumhydroxid in einer Menge, die ausreicht, um den pH-Wert zwischen 7 und 10 einzustellen, gereinigtes Wasser bis zum gewünschten Volumen.

12. Formulierung nach den Ansprüchen 1 bis 9, umfassend Xylotol in einer Menge zwischen 12 und 18 % (p/p), Natriumbicarbonat in einer Menge zwischen 1,5 und 2,5 (p/p), Natriumhyaluronat HMW in einer Menge zwischen 0,02 und 0,08 % (p/p), Glycerin in einer Menge zwischen 10 und 20 % (p/p), Natriumlauroylsarcosinat in einer Menge zwischen 0,5 % und 1,5 % (p/p), Tetranatrium EDTA in einer Menge zwischen 0,02 und 0,08 % (p/p), Trimethylglycin in einer Menge zwischen 8 und 12 % (p/p), Natriumhydroxid in einer Menge, die ausreicht, um den pH-Wert zwischen 7 und 10 einzustellen, gereinigtes Wasser bis zum gewünschten Volumen.

13. Formulierung nach Anspruch 12, umfassend 15 % (p/p) Xylotol, 2 % (p/p) Natriumbicarbonat, 0,05 % (p/p) Natriumhyaluronat HMW, 15 % (p/p) Glycerin, 1 % (p/p) Natriumlauroylsarcosinat, 0,05 % (p/p) Tetranatrium EDTA, 10 % (p/p) Trimethylglycin, Natriumhydroxid in einer Menge, die ausreicht, um den pH-Wert zwischen 7 und 10 einzustellen, gereinigtes Wasser bis zum gewünschten Volumen.

14. Formulierung nach den Ansprüchen 1 bis 13 für die Mundhygiene, insbesondere zur Verminderung oder Vorbeugung von Zahnkaries.

## Revendications

1. Formulation orale liquide aqueuse ayant un pH compris entre pH 7 et 10 et comprenant du xylitol, un sel de bicarbonate et un polymère muco-adhésif, en particulier sélectionné à partir d'un groupe constitué par l'acide polyacrylique, l'hydroxypropylcellulose, l'hydroxypropylméthylcellulose, l'hydroxyethylcellulose, la polyvinylpyrrolidone, les dérivés du polyméthacrylate, l'acide hyaluronique et ses dérivés, le chitosan et ses mélanges, dans laquelle ladite formulation est sous une forme de bain de bouche ou sous une forme nébulisable.

2. Formulation selon la revendication 1, dans laquelle le polymère est l'hyalunorate de sodium.

3. Formulation selon la revendication 1 ou 2, dans laquelle le polymère est présent dans la formulation de l'invention dans une quantité comprise entre 0,02% et 0,1% (p/p).

4. Formulation selon les revendications 1 à 3, dans laquelle le xylitol est contenu dans la formulation dans une quantité comprise entre 8% et 20% (p/p).

5. Formulation selon les revendications 1 à 4, dans laquelle le sel de bicarbonate est contenu dans la formulation dans une quantité comprise entre 1% et 2,5% (p/p).

6. Formulation selon les revendications 1 à 5, comprenant en outre au moins un agent de chélation, un agent tensioactif, en particulier sélectionné parmi le sarcosinate de sodium lauryol, le lauryl sulfate de sodium, lauryl éther sulfate de sodium, poloxamère 407 et polysorbate 20 et au moins un polyol monomère, en particulier sélectionné parmi la glycérine et un glycol.

7. Formulation selon la revendication 6, dans laquelle l'agent de chélation est l'EDTA tétrasodique.

8. Formulation selon la revendication 7 ou 8, dans laquelle ledit agent de chélation est présent dans une quantité comprise entre 0,02% et 1% (p/p).

9. Formulation selon les revendications 6 à 8, dans laquelle ledit polyol monomère est présent dans la formulation dans une quantité comprise entre 10 et 35% (p/p).

10. Formulation selon les revendications 1 à 9, comprenant du xylitol, dans une quantité comprise entre 10 et 15% (p/p), du bicarbonate de sodium, dans une quantité comprise entre 1 et 2% (p/p), de l'hyaluronate de sodium HMW, dans une quantité comprise entre 0,02 et 0,08% (p/p), de la glycérine, dans une quantité comprise entre 15 et 25% (p/p), du butylène glycol, dans une quantité comprise entre 8 et 15% (p/p), du sarcosinate de sodium lauryol, dans une quantité comprise entre 0,2 et 0,8% (p/p), de l'EDTA tétrasodique, dans une quantité comprise entre 0,02 et 0,08% (p/p), de l'hydroxyde de sodium dans une quantité suffisante pour amener le pH entre 7 et 10, et de l'eau purifiée jusqu'au volume désiré.

11. Formulation selon la revendication 10, comprenant 12% (p/p) de xylitol, 1,5% (p/p) de bicarbonate de sodium, 0,05% (p/p) d'hyaluronate de sodium HMW, 20% (p/p) de glycérine, 10% (p/p) de butylène glycol, 0,5% (p/p) de sarcosinate de sodium lauryol, 0,05% d'EDTA tétrasodique (p/p) ; de l'hydroxyde de sodium dans une quantité suffisante pour amener le pH entre 7 et 10, et de l'eau purifiée jusqu'au volume désiré.

12. Formulation selon les revendications 1 à 9, comprenant du xylitol, dans une quantité comprise entre 12 et 18% (p/p), du bicarbonate de sodium, dans une quantité comprise entre 1,5 et 2,5 (p/p), de l'hyaluronate de sodium HMW, dans une quantité comprise entre 0,02 et 0,08% (p/p), de la glycérine, dans une quantité comprise entre 10 et 20% (p/p), du sarcosinate de sodium lauryol dans une quantité comprise entre 0,5% et 1,5% (p/p), de l'EDTA tétrasodique, dans une quantité comprise entre 0,02 et 0,08% (p/p), de la triméthylglycine, dans une quantité comprise entre 8 et 12% (p/p), de l'hydroxyde de sodium dans une quantité suffisante pour amener le pH entre 7 et 10, et de l'eau purifiée jusqu'au volume désiré.

13. Formulation selon la revendication 12, comprenant 15% (p/p) de xylitol, 2% (p/p) de bicarbonate de sodium, 0,05% (p/p) d'hyaluronate de sodium HMW, 15% (p/p) de glycérine, 1% (p/p) de sarcosinate de sodium lauryol, 0,05% (p/p) d'EDTA tétrasodique, 10% (p/p) de triméthylglycine, de l'hydroxyde de sodium dans une quantité suffisante pour amener le pH entre 7 et 10, et de l'eau purifiée jusqu'au volume désiré.

14. Formulation selon les revendications 1 à 13, pour l'utilisation pour l'hygiène buccale, en particulier pour la réduction ou la prévention de la formation de caries dentaires.
